# EUROPÄISCHE PATENTANMELDUNG

(11) **EP 2 184 280 A1**
(43) Veröffentlichungstag der Anmeldung: **12.05.2010**
(21) Anmeldenummer: 10151804.1
(22) Anmeldetag: 12.11.2005
(51) Int. Cl.: C07D 277/20, C07D 213/61, C07D 277/32, C07D 307/14, A01N 47/44, A01N 51/00

(54) **Substituierte Oxyguanidine**

(30) Priorität: 24.11.2004 DE 102004056626
(62) Teilanmeldung aus: 05811067.7
(71) Anmelder: Bayer CropScience AG, 40789 Monheim (DE)
(72) Erfinder: Jeschke, Peter, Dr., 51467 Bergisch Gladbach (DE); Lösel, Peter, Dr., 51371 Leverkusen (DE); Nauen, Ralf, Dr., 40764 Langenfeld (DE); Marczok, Peter, 50739 Köln (DE); Arnold, Christian, Dr., 40764 Langenfeld (DE); Sanwald, Erich, Dr., 24159 Kiel (DE)

(57) **Zusammenfassung**

Die vorliegende Anmeldung betrifft neue substituierte Oxyguanidine der Formel 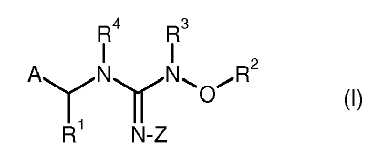 ein Verfahren zu ihrer Herstellung und ihre Verwendung zur Bekämpfung von tierischen Schädlingen, vor allem von Arthropoden, insbesondere von Insekten.

## Beschreibung

Die vorliegende Anmeldung betrifft neue substituierte Oxyguanidine, ein Verfahren zu ihrer Herstellung und ihre Verwendung zur Bekämpfung von tierischen Schädlingen, vor allem von Arthropoden, insbesondere von Insekten.

Eine Reihe von N-Alkyl-N'-nitro-guanidinen ist bereits als insektizid wirksame Verbindungen bekannt geworden (vgl. EP-375907, EP-376279, EP-425978, EP-483062).

Mit der Verbindung N-Methoxy-N'-nitro-N"-(tetrahydro-3-furanyl-methyl)-guanidin ist auch ein Beispiel für ein N-Alkoxy-N'-nitro-guanidin bekannt geworden (vgl. EP-649845).

Es wurden nun neue Verbindungen der Formel (I) gefunden, 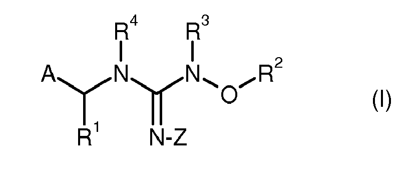 in welcher
- A: für jeweils gegebenenfalls substituiertes Aryl oder Heteroaryl steht und - für den Fall, dass Z für Cyano steht - auch für gegebenenfalls substituiertes Tetrahydrofuryl steht,
- R¹: für Wasserstoff oder Alkyl steht,
- R²: für Wasserstoff oder Alkyl steht,
- R³: für Wasserstoff oder Alkyl steht,
- R⁴: für Wasserstoff, Alkyl oder Cycloalkyl steht, und
- Z: für Cyano oder Nitro steht.

Weiter wurde gefunden, dass man die neuen substituierten Oxyguanidine der Formel (I) erhält, wenn man Isothioharnstoffe der Formel (II) 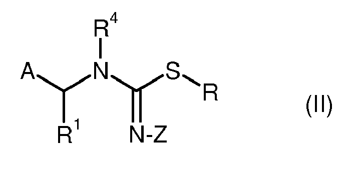 in welcher
- A, R¹, R⁴ und Z: die oben angegebene Bedeutung haben und
- R: für Alkyl steht,
mit Oxyaminen der Formel (III) 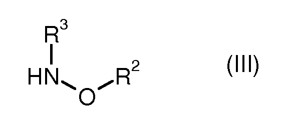 in welcher
R² und R³ die oben angebenene Bedeutung haben,
- oder mit Säureaddukten der Verbindungen der Formel (II), wie beispielsweise deren Hydrochloriden -
gegebenenfalls in Gegenwart eines basischen Reaktionshilfsmittel und gegebenenfalls in Gegenwart eines Verdünnungsmittel umsetzt.

Schließlich wurde gefunden, dass die neuen Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die Verbindungen der Formel (I) können gegebenenfalls in Abhängigkeit von der Art der Substituenten als geometrische und/oder als optisch aktive Isomere oder entsprechende Isomerengemische in unterschiedlicher Zusammensetzung vorliegen. Die Erfindung betrifft sowohl die reinen Isomeren als auch die Isomerengemische.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im Folgenden erläutert.
- A: steht bevorzugt für gegebenenfalls durch Halogen, Cyano, Nitro, C₁-C₄-Alkyl, C₁-C₄-Halogenalkyl, C₁-C₄-Alkoxy oder C₁-C₄-Halogenalkoxy substituiertes Phenyl.
- A: steht bevorzugt auch für Pyrazolyl, 1,2,4-Triazolyl, Oxazolyl, Isoxazolyl, Thiazolyl, Isothiazolyl, 1,2,5-Thiadiazolyl, Pyridyl, Pyrazinyl oder Pyrimidinyl, welche gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist) oder C₁-C₂-Alkylsulfonyl (welches gegebenenfalls durch Fluor und/oder Chlor substituiert ist) substituiert sind.
- A: steht bevorzugt - für den Fall, dass Z für Cyano steht - auch für gegebenenfalls durch C₁-C₄-Alkyl substituiertes Tetrahydrofuryl.
- R¹: steht bevorzugt für Wasserstoff oder Methyl, Ethyl oder n-Propyl oder iso-Propyl.
- R²: steht bevorzugt für Wasserstoff, Methyl, Ethyl, n-Propyl oder n-Butyl.
- R³: steht bevorzugt für Wasserstoff, Methyl oder Ethyl.
- R⁴: steht bevorzugt für Wasserstoff, Methyl, Ethyl, n- oder i-Propyl, Cyclopropyl, Cyclobutyl, Cyclopentyl oder Cyclohexyl.
- Z: steht bevorzugt für Cyano oder Nitro.
- A: steht besonders bevorzugt für Thiazolyl oder Pyridyl welche gegebenenfalls substituiert sind durch Halogen (insbesondere Chlor) oder C₁-C₃-Alkyl (insbesondere Methyl).
- R¹: steht besonders bevorzugt für Wasserstoff oder Methyl.
- R²: steht besonders bevorzugt für Wasserstoff, Methyl, Ethyl oder n-Propyl.
- R³: steht besonders bevorzugt für Wasserstoff oder Methyl.
- R⁴: steht besonders bevorzugt für Wasserstoff, Methyl, Ethyl oder Cyclopropyl.
- Z: steht besonders bevorzugt für Cyano oder Nitro.
- A: steht ganz besonders bevorzugt für 2-Chlor-pyridin-5-yl oder 2-Chlor-1,3-thiazol-5-yl.
- R¹: steht ganz besonders bevorzugt für Wasserstoff.
- R²: steht ganz besonders bevorzugt für Methyl.
- R²: steht ganz besonders bevorzugt auch für Ethyl.
- R³: steht ganz besonders bevorzugt für Wasserstoff.
- R⁴: steht ganz besonders bevorzugt für Wasserstoff.
- Z: steht ganz besonders bevorzugt für Nitro.

In einer hervorgehobenen Gruppe von Verbindungen der Formel (I) steht A für 2-Chlor-pyridin-5-yl

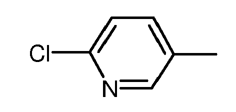

In einer weiteren hervorgehobenen Gruppe von Verbindungen der Formel (I) steht A für 2-Chlor-1,3-thiazol-5-yl

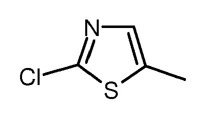

Erfindungsgemäß bevorzugt werden die Verbindungen der Formel (I), bei welchen die Kombinationen der vorstehend als bevorzugt aufgeführten Bedeutungen vorliegen.

Erfindungsgemäß besonders bevorzugt werden die Verbindungen der Formel (I), bei welchen die Kombinationen der vorstehend als besonders bevorzugt aufgeführten Bedeutungen vorliegen.

Erfindungsgemäß ganz besonders bevorzugt werden die Verbindungen der Formel (I), bei welchen die Kombinationen der vorstehend als ganz besonders bevorzugt aufgeführten Bedeutungen vorliegen.

In den oben und nachstehend aufgeführten Restedefinitionen sind Kohlenwasserstoffreste, wie Alkyl - auch in Verbindung mit Heteroatomen wie Alkoxy - soweit möglich jeweils geradkettig oder verzweigt.

Verwendet man beispielsweise N-Nitro-N'-(2-chlor-pyridin-5-yl-methyl)-S-methyl-isothioharnstoff und O-Methyl-hydroxylamin als Ausgangsstoffe, so kann der Reaktionsablauf beim erfindungsgemäßen Verfahren durch das folgende Formelschema skizziert werden:

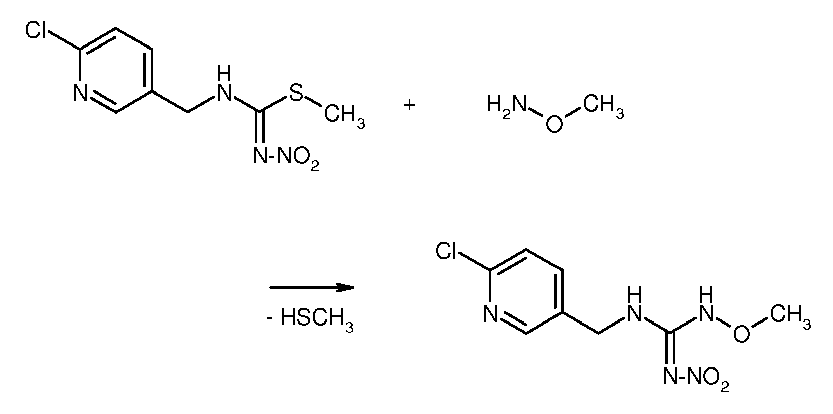

Die beim erfindungsgemäßen Verfahren zur Herstellung der Verbindungen der allgemeinen Formel (I) als Ausgangsstoffe zu verwendenden Isothioharnstoffe sind durch die Formel (II) allgemein definiert. In der allgemeinen Formel (II) haben A und R¹ vorzugsweise bzw. insbesondere diejenigen Bedeutungen, die bereits oben im Zusammenhang mit der Beschreibung der erfindungsgemäßen Verbindungen der allgemeinen Formel (I) als bevorzugt bzw. als besonders bevorzugt für A und R¹ angegeben worden sind; R steht für Alkyl, vorzugsweise für Alkyl mit 1 bis 4 Kohlenstoffatomen, insbesondere für Methyl oder Ethyl.

Die Ausgangsstoffe der allgemeinen Formel (II) sind bekannt und/oder können nach an sich bekannten Verfahren hergestellt werden (vgl. EP-452782).

Die beim erfindungsgemäßen Verfahren zur Herstellung der Verbindungen der allgemeinen Formel (I) weiter als Ausgangsstoffe zu verwendenden Oxyamine sind durch die Formel (III) allgemein definiert. In der allgemeinen Formel (III) haben R² und R³ vorzugsweise bzw. insbesondere diejenigen Bedeutungen, die bereits oben im Zusammenhang mit der Beschreibung der erfindungsgemäßen Verbindungen der allgemeinen Formel (I) als bevorzugt bzw. als besonders bevorzugt für R² und R³ angegeben worden sind.

Die Ausgangsstoffe der allgemeinen Formel (III) sind bekannte organische Synthesechemikalien.

Als basische Reaktionshilfsmittel zur Durchführung des erfindungsgemäßen Verfahrens können alle geeigneten anorganischen oder organischen Säureakzeptoren eingesetzt werden. Hierzu gehören vorzugsweise Alkalimetall- und Erdalkalimetallverbindungen sowie basische Stickstoffverbindungen, insbesondere Alkylamine.

Beispielhaft seien dafür erwähnt die Hydroxide, Oxide und Carbonate des Lithiums, Natriums, Kaliums, Magnesiums, Calciums und Bariums, ferner weitere basische Verbindungen wie Amidinbasen oder Guanidinbasen wie 7-Methyl-1,5,7-triazabicyclo(4.4.0)-dec-5-en (MTBD), Diaza-bicyclo(4.3.0)nonen (DBN), Diazabicyclo(2.2.2)octan (DABCO), 1,8-Diaza-bicyclo(5.4.0)undecen (DBU), Cyclohexyl-tetrabutylguanidin (CyTBG), Cyclohexyltetramethylguanidin (CyTMG), N,N,N,N-Tetramethyl-1,8-naphthalindiamin, Pentamethylpiperidin, tertiäre Amine wie Triethylamin, Trimethylamin, Tribenzylamin, Triisobutylamin, Tributylamin, Tribenzylamin, Tricyclohexylamin, Triamylamin, Trihexylamin, N,N-Dimethylanilin, N,N-Dimethyl-toluidin, N,N-Dimethyl-p-aminopyridin, N-Methyl-pyrrolidin, N-Methyl-piperidin, N-Methyl-imidazol, N-Methyl-pyrrol, N-Methyl-morpholin, N-Methyl-hexamethylenimin, Pyridin, 4-Pyrrolidino-pyridin, 4-Dimethylamino-pyridin, Chinolin, α-Picolin, β-Picolin, Isochinolin, Pyrimidin, Acridin, N,N,N',N'-Tetramethylendiamin, N,N,N',N'-Tetraethylendiamin, Chinoxalin, N-Propyl-diisopropylamin, N-Ethyl-diisopropylamin, N,N'-Dimethyl-cyclohexylamin, 2,6-Lutidin, 2,4-Lutidin oder Triethylendiamin.

Vorzugsweise finden tertiäre Amine, insbesondere Trialkylamine wie Triethylamin, N,N-Diisopropylamin, N-Propyl-diisopropylamin, N,N'-Dimethyl-cyclohexylamin oder N-Methyl-morpholin, Verwendung.

Als Verdünnungsmittel zur Durchführung des erfindungsgemäßen Verfahrens kommen alle unter den Reaktionsbedingungen inerten Lösungsmittel in Frage.

Als Beispiele sind zu nennen: Halogenkohlenwasserstoffe, insbesondere Chlorkohlenwasserstoffe wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether wie Ethylpropylether, Methyl-tert-butylether, n-Butylether, Anisol, Phenethol, Cyclohexylmethylether, Dimethylether, Diethylether, Dipropylether, Diisopropylether, Di-n-propylether, Diisobutylether, Diisoamylether, Ethylenglycoldimethylether, Tetrahydrofuran, Dioxan, Dichlordiethylether und Polyether des Ethylenoxids und/oder Propylenoxids; Amine wie Trimethyl-, Triethyl-, Tripropyl-, Tributylamin, N-Methyl-morpholin, Pyridin und Tetramethylendiamin, Nitrokohlenwasserstoffe wie Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril sowie Verbindungen wie Tetrahydrothiophenoxid und Dimethylsulfoxid, Tetramethylsulfoxid, Dipropylsulfoxid, Benzylmethylsulfoxid, Diisobutylsulfoxid, Dibutylsulfoxid, Diisoamylsulfoxid; Sulfone wie Dimethyl-, Dieethyl-, Dipropyl-, Dibutyl-, Diphenyl-, Dihexyl-, Methylhexyl-, Ethylpropyl-, Ethylisobutyl- und Pentamethylensulfon; aliphatische, cycloaliphatische oder aromatische Kohlenwasserstoffe, beispielsweise sogenannte White Spirits mit Komponenten mit Siedepunkten im Bereich beispielsweise von 40°C bis 250°C, Cymol, Benzinfraktionen innerhalb eines Siedeintervalls von 70°C bis 190°C, Cyclohexan, Methylcyclohexan, Petrolether, Ligroin, Octan, Benzol, Toluol, Chlorbenzol, Brombenzol, Nitrotoluol, Xylol; Ester wie Methyl-, Ethyl-, Butyl-, Isobutylacetat sowie Dimethyl-, Dibutyl-, Ethylencarbonat; Amide wie Hexamethylenphosphorsäuretriamid, Formamid, N-Methylformamid, N,N-Dimethyl-formamid, N,N-Dipropyl-formamid, N,N-Dibutyl-formamid, N-Methyl-pyrrolidin, N-Methyl-caprolactam, 1,3-Dimethyl-3,4,5,6-tetrahydro-2(1H)pyrimidin, Octylpyrrolidin, Octylcaprolactam, 1,3-Dimethyl-2-imidazolindion, N-Formyl-piperidin, N,N'-1,4-Diformyl-piperazin; Ketone wie Aceton, Acetophenon, Methylethylketon, Methylbutylketon, Wasser.

Selbstverständlich kann man das erfindungsgemäße Verfahren auch in Gemischen der genannten Lösungs- und Verdünnungsmittel durchführen.

Bevorzugte Verdünnungsmittel sind jedoch Alkohole, wie beispielsweise Methanol oder Ethanol, gegebenenfalls im Gemisch mit Wasser.

Die Reaktionstemperaturen können bei der Durchführung des erfindungsgemäßen Verfahrens in einem größeren Bereich variiert werden. Im Allgemeinen arbeitet man bei Temperaturen zwischen -50°C und +150°C, vorzugsweise zwischen -30°C und +100°C, insbesondere zwischen -15°C und +80°C.

Das erfindungsgemäße Verfahren wird im Allgemeinen unter Normaldruck durchgeführt. Es ist jedoch auch möglich, das erfindungsgemäße Verfahren unter erhöhtem oder vermindertem Druck - im Allgemeinen zwischen 0,1 bar und 15 bar - durchzuführen.

Zur Durchführung des erfindungsgemäßen Verfahrens werden die Ausgangsstoffe im Allgemeinen in angenähert äquimolaren Mengen eingesetzt. Es ist jedoch auch möglich, eine der Komponenten in einem größeren Überschuss zu verwenden. Die Umsetzung wird im Allgemeinen in einem geeigneten Verdünnungsmittel in Gegenwart eines Reaktionshilfsmittels, gegebenenfalls auch unter einer Schutzgas-Atmosphäre (z.B. unter Stickstoff, Argon oder Helium) durchgeführt und das Reaktionsgemisch wird im Allgemeinen mehrere Stunden bei der erforderlichen Temperatur gerührt. Die Aufarbeitung wird nach üblichen Methoden durchgeführt (vgl. die Herstellungsbeispiele).

Alternativ kann die Herstellung der erfindungsgemäßen Verbindungen der Formel (I) auch aus Verbindungen der allgemeinen Formel (IV) - in welcher R⁴ für Wasserstoff steht - nach literaturbekannten Methoden erfolgen; beispielsweise kann das Hexahydro-1,3,5-triazin-System in Gegenwart von sauren Reaktionshilfsmitteln (vgl. Tetrahedron Lett. 41, 7187-7191, 2000) oder basischen Reaktionshilfsmitteln, in Gegenwart von Harnstoff (vgl. WO-98/42690) oder primären Aminen (vgl. DE-19806469) entsprechend folgenden Schema gespalten werden.

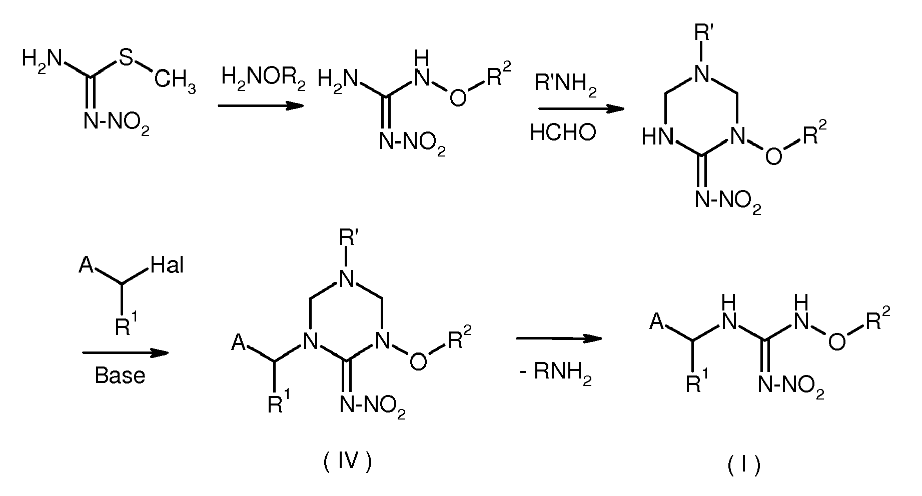

Die hierbei als Ausgangsstoffe benötigten Verbindungen sind durch die Formel (IV) allgemein definiert. In der Formel (IV) stehen vorzugsweise für A, R¹ und R² für diejenigen Reste, die bereits im Zusammenhang mit der Beschreibung der erfindungsgemäßen Stoffe der Formel (I) als bevorzugte Substituenten genannt wurden. Weiterhin steht in Formel (IV) der Rest R' für eine Alkyl oder Arylalkylgruppe.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..
Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.
Aus der Klasse der Bivalva z.B. Dreissena spp..
Aus der Ordnung der Chilopoda z.B. Geophilus spp., Scutigera spp..
Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..
Aus der Ordnung der Collembola z.B. Onychiurus armatus.
Aus der Ordnung der Dermaptera z.B. Forficula auricularia.
Aus der Ordnung der Diplopoda z.B. Blaniulus guttulatus.
Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.
Aus der Klasse der Gastropoda z.B. Arion spp., Biomphalaria spp., Bulinus spp., Deroceras spp., Galba spp., Lymnaea spp., Oncomelania spp., Succinea spp..
Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.
Weiterhin lassen sich Protozoen, wie Eimeria, bekämpfen.
Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.
Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp.,
Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.
Aus der Ordnung der Hymenoptera z.B. Diprion spp., Hoplocampa spp., Lasius spp., Monomorium pharaonis, Vespa spp..
Aus der Ordnung der Isopoda z.B. Armadillidium vulgare, Oniscus asellus, Porcellio scaber.
Aus der Ordnung der Isoptera z.B. Reticulitermes spp., Odontotermes spp..
Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..
Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.
Aus der Ordnung der Siphonaptera z.B. Ceratophyllus spp., Xenopsylla cheopis.
Aus der Ordnung der Symphyla z.B. Scutigerella immaculata.
Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..
Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen der Formel (I) zeichnen sich insbesondere durch starke Wirkung gegen Blattläuse (z.B. Aphis gossypii und Myzus (persicae) und gegen die Weiße Fliege (Bemisia tabaci) aus.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-FettalkoholEther, z.B. Alkylaryl-polyglykolether, Alkylsulfonate, Alkylsulfate, Arylsulfonate sowie Einweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, AlkylAryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im Allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Besonders günstige Mischpartner sind z.B. die folgenden:
Fungizide:
   Inhibitoren der Nucleinsäure Synthese
      Benalaxyl, Benalaxyl-M, Bupirimat, Chiralaxyl, Clozylacon, Dimethirimol, Ethirimol, Furalaxyl, Hymexazol, Metalaxyl, Metalaxyl-M, Ofurace, Oxadixyl, Oxolinsäure
   Inhibitoren der Mitose und Zellteilung
      Benomyl, Carbendazim, Diethofencarb, Fuberidazole, Pencycuron, Thiabendazol, Thiophanat-methyl, Zoxamid
   Inhibitoren der Atmungskette Komplex I
      Diflumetorim
   Inhibitoren der Atmungskette Komplex II
      Boscalid, Carboxin, Fenfuram, Flutolanil, Furametpyr, Mepronil, Oxycarboxin, Penthiopyrad, Thifluzamid
   Inhibitoren der Atmungskette Komplex III
      Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Picoxystrobin
   Entkoppler
      Dinocap, Fluazinam
   Inhibitoren der ATP Produktion
      Fentinacetat, Fentinchlorid, Fentinhydroxid, Silthiofam
   Inhibitoren der Aminosäure- und Proteinbiosynthese
      Andoprim, Blasticidin-S, Cyprodinil, Kasugamycin, Kasugamycinhydrochlorid Hydrat, Mepanipyrim, Pyrimethanil
   Inhibitoren der Signal-Transduktion
      Fenpiclonil, Fludioxonil, Quinoxyfen
   Inhibitoren der Fett- und Membran Synthese
      Chlozolinat, Iprodion, Procymidon, Vinclozolin
      Ampropylfos, Kalium-Ampropylfos, Edifenphos, Iprobenfos (IBP), Isoprothiolan, Pyrazophos
      Tolclofos-methyl, Biphenyl
      Iodocarb, Propamocarb, Propamocarb hydrochlorid
   Inhibitoren der Ergosterol Biosynthese
      Fenhexamid,
      Azaconazol, Bitertanol, Bromuconazol, Cyproconazol, Diclobutrazol, Difenoconazol, Diniconazol, Diniconazol-M, Epoxiconazol, Etaconazol, Fenbuconazol, Fluquinconazol, Flusilazol, Flutriafol, Furconazol, Furconazol-cis, Hexaconazol, Imibenconazol, Ipconazol, Metconazol, Myclobutanil, Paclobutrazol, Penconazol, Propiconazol, Prothioconazol, Simeconazol, Tebuconazol, Tetraconazol, Triadimefon, Triadimenol, Triticonazol, Uniconazol, Voriconazol, Imazalil, Imazalilsulfat, Oxpoconazol, Fenarimol, Flurprimidol, Nuarimol, Pyrifenox, Triforin, Pefurazoat, Prochloraz, Triflumizol, Viniconazol,
      Aldimorph, Dodemorph, Dodemorphacetat, Fenpropimorph, Tridemorph, Fenpropidin, Spiroxamin,
      Naftifin, Pyributicarb, Terbinafin
   Inhibitoren der Zellwand Synthese
      Benthiavalicarb, Bialaphos, Dimethomorph, Flumorph, Iprovalicarb, Polyoxins, Polyoxorim, Validamycin A
   Inhibitoren der Melanin Biosynthese
      Capropamid, Diclocymet, Fenoxanil, Phtalid, Pyroquilon, Tricyclazol
   Resistenzinduktion
      Acibenzolar-S-methyl, Probenazol, Tiadinil
   Multisite
      Captafol, Captan, Chlorothalonil, Kupfersalze wie: Kupferhydroxid, Kupfernaphthenat, Kupferoxychlorid, Kupfersulfat, Kupferoxid, Oxin-Kupfer und Bordeaux Mischung, Dichlofluanid, Dithianon, Dodin, Dodin freie Base, Ferbam, Fluorofolpet, Guazatin, Guazatinacetat, Iminoctadin, Iminoctadinalbesilat, Iminoctadintriacetat, Mankupfer, Mancozeb, Maneb, Metiram, Metiram Zink, Propineb, Schwefel und Schwefelpräparate enthaltend Calciumpolysulphid, Thiram, Tolylfluanid, Zineb, Ziram
   Unbekannter Mechanismus
      Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ethaboxam, Ferimzon, flumetover, Flusulfamid, Fluopicolid, Fluoroimid, Hexachlorobenzol, 8-Hydroxychinolinsulfat, Irumamycin, Methasulphocarb, Metrafenon, Methyl Isothiocyanat, Mildiomycin, Natamycin, Nickel dimethyldithiocarbamat, Nitrothal-isopropyl, Octhilinon, Oxamocarb, Oxyfenthiin, Pentachlorophenol und Salze, 2-Phenylphenol und Salze, Piperalin, Propanosin -Natrium, Proquinazid, Pyrrolnitrin, Quintozen, Tecloftalam, Tecnazen, Triazoxid, Trichlamid, Zarilamid und 2,3,5,6-Tetrachlor-4-(methylsulfonyl)-pyridin, N-(4-Chlor-2-nitrophenyl)-N-ethyl-4-methyl-benzenesulfonamid, 2-Amino-4-methyl-N-phenyl-5-thiazolecarboxamid, 2-Chlor-N-(2,3-dihydro-1,1,3-trimethyl-1H-inden-4-yl)-3-pyridin-carboxamid, 3-[5-(4-Chlorphenyl)-2,3-dimethylisoxazolidin-3-yl]pyridin, cis-1-(4-Chlorphenyl)-2-(1H-1,2,4-triazol-1-yl)-cycloheptanol, 2,4-Dihydro-5-methoxy-2-methyl-4-[[[[1-[3-(trifluoromethyl)-phenyl]-ethyliden]-amino]-oxy]-methyl]-phenyl]-3H-1,2,3-triazol-3-on (185336-79-2), Methyl 1-(2,3-dihydro-2,2-dimethyl-1H-inden-1-yl)-1H-imidazole-5-carboxylat, 3,4,5-Trichlor-2,6-pyridindicarbonitril, Methyl 2-[[[cyclopropyl[(4-methoxyphenyl) imino]methyl]thio]methyl]-.alpha.-(methoxymethylen)- benzacetat, 4-Chlor-alpha-propinyloxy-N-[2-[3-methoxy-4-(2-propinyloxy)phenyl]ethyl]-benzacetamide, (2S)-N-[2-[4-[[3-(4-chlorophenyl)-2-propinyl]oxy]-3-methoxyphenyl]ethyl]- 3-methyl-2-[(methylsulfonyl)amino]-butanamid, 5-Chlor-7-(4-methylpiperidin-1-yl)-6-(2,4,6-trifluoro-phenyl)[1,2,4]triazolo[1,5-a]pyrimidin, 5-Chlor-6-(2,4,6-trifluorophenyl)-N-[(1R)-1,2,2-trimethylpropyl][1,2,4]triazolo[1,5-a]pyrimidin-7-amin, 5-Chlor-N-[(1R)-1,2-dimethylpropyl]-6-(2,4,6-trifluorophenyl) [1,2,4]triazolo[1,5-a]pyrimidin-7-amine, N-[1-(5-Brom-3-chloropyridin-2-yl)ethyl]-2,4-dichloronicotinamid, N-(5-Brom-3-chlorpyridin-2-yl)methyl-2,4-dichlornicotinamid, 2-Butoxy-6-iod-3-propyl-benzopyranon-4-on, N-{(Z)-[(cyclopropyl-methoxy) imino][6-(difluormethoxy)-2,3-difluorphenyl]methyl}-2-benzacetamid, N-(3-Ethyl-3,5,5-trimethyl-cyclohexyl)-3-formylamino-2-hydroxy-benzamid, 2-[[[[1-[3(1fluor-2-phenylethyl)oxy] phenyl] ethyliden]amino]oxy]methyl]-alpha-(methoxyimino)-N-methyl-alphaE-benzacetamid, N-{2-[3-Chlor-5-(triiluormethyl)pyridin-2-yl]ethyl}-2-(trifluoromethyl)benzamid, N-(3',4'-dichlor-5-fluorbiphenyl-2-yl)-3-(difluormethyl)-1-methyl-1H-pyrazol-4-carboxamid, N-(6-Methoxy-3-pyridinyl)-cyclopropan carboxamid, 1-[(4-Methoxyphenoxy)methyl]-2,2-dimethylpropyl-1H-imidazol-1-carbonsäure, O-[1-[(4-Methoxyphenoxy)methyl]-2,2-dimethylpropyl]-1H-imidazol-1-carbothioic acid, 2-(2-{[6-(3-Chlor-2-methylphenoxy)-5-fluorpyrimidin-4-yl]oxy}phenyl)-2-(methoxyimino)-N-methylacetamid
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Acetylcholinesterase (AChE) Inhibitoren
      Carbamate,
      zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
      Organophosphate,
      zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos(-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/- ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion
   Natrium-Kanal-Modulatoren / Spannungsabhängige Natrium-Kanal-Blocker
      Pyrethroide,
      zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda-Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R- isomer), Tralomethrin, Transfluthrin, ZXI 8901, Pyrethrins (pyrethrum)
      DDT
      Oxadiazine,
      zum Beispiel Indoxacarb
   Acetylcholin-Rezeptor-Agonisten/-Antagonisten
      Chloronicotinyle,
      zum Beispiel Acetamiprid, Clothianidin, Dinotefuran, Imidacloprid, Nitenpyram, Nithiazine, Thiacloprid, Thiamethoxam
      Nicotine, Bensultap, Cartap
   Acetylcholin-Rezeptor-Modulatoren
      Spinosyne,
      zum Beispiel Spinosad
   GABA-gesteuerte Chlorid-Kanal-Antagonisten
      Organochlorine,
      zum Beispiel Camphechlor, Chlordane, Endosulfan, Gamma-HCH, HCH, Heptachlor, Lindane, Methoxychlor
      Fiprole,
      zum Beispiel Acetoprole, Ethiprole, Fipronil, Pyrafluprole, Pyriprole, Vaniliprole
   Chlorid-Kanal-Aktivatoren
      Mectine,
      zum Beispiel Avermectin, Emamectin, Emamectin-benzoate, Ivermectin, Milbemycin
   Juvenilhormon-Mimetika,
      zum Beispiel Diofenolan, Epofenonane, Fenoxycarb, Hydroprene, Kinoprene, Methoprene, Pyriproxifen, Triprene
   Ecdysonagonisten/disruptoren
      Diacylhydrazine,
      zum Beispiel Chromafenozide, Halofenozide, Methoxyfenozide, Tebufenozide
   Inhibitoren der Chitinbiosynthese
      Benzoylharnstoffe,
      zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
      Buprofezin
      Cyromazine
   Inhibitoren der oxidativen Phosphorylierung, ATP-Disruptoren
      Diafenthiuron
      Organozinnverbindungen,
      zum Beispiel Azocyclotin, Cyhexatin, Fenbutatin-oxide
   Entkoppler der oxidativen Phoshorylierung durch Unterbrechung des H-Protongradienten
      Pyrrole,
      zum Beispiel Chlorfenapyr
      Dinitrophenole,
      zum Beispiel Binapacyrl, Dinobuton, Dinocap, DNOC
   Seite-I-Elektronentransportinhibitoren
      METI's,
      zum Beispiel Fenazaquin, Fenpyroximate, Pyrimidifen, Pyridaben, Tebufenpyrad, Tolfenpyrad
      Hydramethylnon
      Dicofol
   Seite-II-Elektronentransportinhibitoren
      Rotenone
   Seite-III-Elektronentransportinhibitoren
      Acequinocyl, Fluacrypyrim
   Mikrobielle Disruptoren der Insektendarmmembran
      Bacillus thuringiensis-Stämme
   Inhibitoren der Fettsynthese
      Tetronsäuren,
      zum Beispiel Spirodiclofen, Spiromesifen
      Tetramsäuren,
      zum Beispiel Spirotetramat (CAS-Reg.-No.: 203313-25-1) und 3-(2,5-Dimethylphenyl)-8-methoxy-2-oxo-1-azaspiro[4.5]dec-3-en-4-yl ethyl carbonate (alias: Carbonic acid, 3-(2,5-dimethylphenyl)-8-methoxy-2-oxo-1-azaspiro[4.5]dec-3-en-4-yl ethyl ester, CAS-Reg.-No.: 382608-10-8)
      Carboxamide,
      zum Beispiel Flonicamid
      Oktopaminerge Agonisten,
      zum Beispiel Amitraz
   Inhibitoren der Magnesium-stimulierten ATPase,
      Propargite
      Benzoesäuredicarboxamide,
      zum Beispiel Flubendiamide
      Nereistoxin-Analoge,
      zum Beispiel Thiocyclam hydrogen oxalate, Thiosultap-sodium
   Biologika, Hormone oder Pheromone
      Azadirachtin, Bacillus spec., Beauveria spec., Codlemone, Metarrhizium spec., Paecilomyces spec., Thuringiensin, Verticillium spec.
   Wirkstoffe mit unbekannten oder nicht spezifischen Wirkmechanismen
      Begasungsmittel,
      zum Beispiel Aluminium phosphide, Methyl bromide, Sulfuryl fluoride
      Fraßhemmer,
      zum Beispiel Cryolite, Flonicamid, Pymetrozine
      Milbenwachstumsinhibitoren,
      zum Beispiel Clofentezine, Etoxazole, Hexythiazox
      Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp.
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.
Aus der Ordnung der Siphonapterida z.B. Pulex spp., Ctenocephalides spp., Xenopsylla spp., Ceratophyllus spp.
Aus der Ordnung der Heteropterida z.B. Cimex spp., Triatoma spp., Rhodnius spp., Panstrongylus spp.
Aus der Ordnung der Blattarida z.B. Blatta orientalis, Periplaneta americana, Blattela germanica, Supella spp.
Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp.
Aus der Ordnung der Chilopoda z.B. Geophilus spp.
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele:

### Beispiel I-1

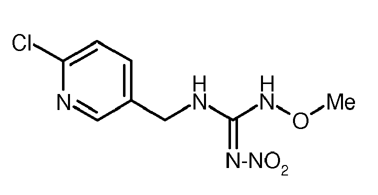

2,60 g (10,0 mMol) N-Nitro-N'-(2-chlor-pyrid-5-yl-methyl)-S-methyl-isothioharnstoff und 0,84 g (10,0 mMol) O-Methyl-hydroxylamin-hydrochlorid werden mit 1,10 g (11 mMol) Triethylamin in einem Gemisch aus 60 ml Ethanol / Wasser (1:1) ca. 18 Stunden bei 50°C bis 55°C gerührt. Anschließend wird der Reaktionsansatz unter vermindertem Druck eingeengt und mittels Säulenchromatographie aufgetrennt.

Man erhält 1,4 g (54 % der Theorie) N-Methoxy-N'-(2-chlor-pyrid-5-yl-methyl)-nitroguanidin.
C₈H₁₀ClN₅O₃ (359,6)
LC-MS mz (%) = 260 (MH⁺, 100).
¹H-NMR (600 MHz, DMSO-d₆, δ) = 3,72 (3H, -O-C**H**₃); 4,39 (2H, -C**H**₂-Cl-Py); 7,51; 7,78; 8,35 (3x 1H, Cl-Py-C**H**-); 8,49; 11,82 (2x 1H, -NH) ppm.
¹³C- mit ¹H-Entkopplung (CPD) und ¹H- mit ¹³C-Korrelation (HMQC, HMBC; 600 MHz, DMSO-d₆, δ) = 41,1 (-NH-CH₂-Cl-Py); 64,1 (-O-CH₃); 124,2 (Cl-Py-CH-); 134,1 (Cl-Py-C-); 139,1 (Cl-Py-CH-); 149,2 (Cl-Py-CH-); 149,1 (Cl-Py-C-Cl); 156,6 (-C=N-NO₂) ppm.
¹H- / ¹⁵N-Korrelation (HMBC, 600 MHz, DMSO-d₆, δ) = -209,9 (-**N**H-O-); -295,2 (-**N**H-CH₂-); - 74,1 (Cl-Py-**N**-) ppm.

Analog zu Herstellungsbeispiel 1 und entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

**Tabelle 1**

| | | | | | | | |
|---|---|---|---|---|---|---|---|
| 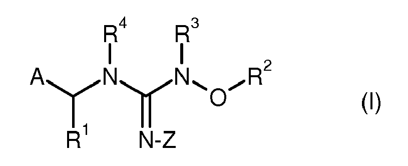 | | | | | | | |

| **Bsp.- Nr.** | **A** | **R¹** | **R²** | **R³** | **R⁴** | **Z** | **Physikal. Daten** |
|---|---|---|---|---|---|---|---|
| I-2 | 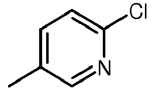 | H | CH₂CH₃ | H | H | NO₂ | ¹³C-NMR (CDCl₃, 400 MHz); 13,3 (CH₃); 41,7 (HN-CH₂); 72,8 (O-CH₂); 124,3, 138,8; 149,1 (=CH-, hetaryl); 150,7 (Cl-C=, hetaryl); 132,1 (-C=, hetaryl); 157,5 (C=N-NO₂ ppm |
| I-3 | 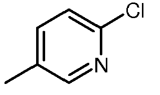 | H | CH₂CH₂CH₂CH₃ | H | H | NO₂ | logP = 2,06 (pH = 2) |
| I-4 | 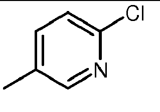 | H | H | H | H | NO₂ | ¹³C-NMR (DMF-d₇, 400 MHz); 41,6 (HN-CH₂); 124,6, 139,6; 149,9 (=CH-; hetaryl); 149,9 (Cl-C=, hetaryl); 135,1 (-C=, hetaryl); 158,9 (C=N-NO₂ ppm |
| I-5 | 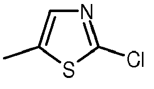 | H | CH₃ | H | H | NO₂ | logP = 1,13 (pH = 2) |
| I-6 | 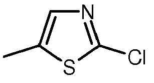 | H | CH₂CH₃ | H | H | NO₂ | logP = 1,42 (pH = 2) |
| I-7 | 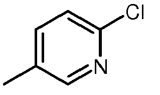 | H | CH₃ | CH₃ | H | NO₂ | logP = 0,67 (pH = 2) |
| I-8 | 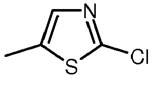 | H | CH₃ | H | CH₃ | NO₂ | logP = 0,75 (pH = 2) |
| I-9 | 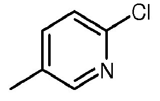 | H | CH₃ | H | CH₃ | NO₂ | logP = 0,96 (pH = 2) |

### Beispiel (I-10)

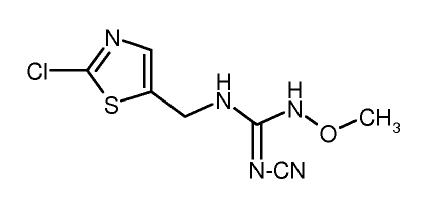

### N-Methoxyamino-N'-(2-chlor-1,3-thiazol-5-yl)-cyanoguanidin

169,2 mg (2,0 mMol) O-Methylhydroxylamin-hydrochlorid und 500 mg (2,0 mMol) S-Methyl-N-cyan-N'-(2-chlor-1,3-thiazol-5-yl)-isothioharnstoff werden in 10 ml Ethanol vorgelegt und mit 0,31 ml (2,2 mMol) Triethylamin und 10,0 ml Wasser versetzt. Anschließend wird der Reaktionsansatz drei Tage bei 40°C, zwei weitere Tage bei 60°C und einen Tag bei 80°C gerührt. Nach Beendigung der Reaktion wird das Ethanol im Vakuum abdestilliert und der gebildete Rückstand dreimal gegen Essigsäureethylester geschüttelt. Die organische Phase wird nach dem Trocknen im Vakuum eingeengt und der zurückgebliebene Rückstand mittels präparativer HPLC gereinigt. Man erhält 16,6 mg (3,1 %) N-Methoxyamino-N'-(2-chlor-1,3-thioazol-5-yl)-cyanoguanidin.
C₇H₈ClN₅OS (245,7)
LC-MS m/z (%) = 247 (MH⁺)

### Beispiel (I-11)

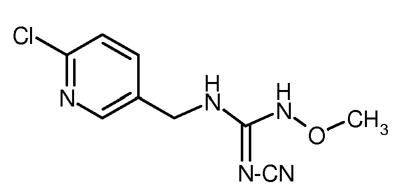

### N-Methoxyamino-N'-(6-chlorpyrid-3-yl)-cyanoguanidin

169,2 mg (2,0 mMol) O-Methylhydroxylamin-hydrochlorid und 2,0 g (8,3 mMol) S-Methyl-N-cyan-N'-(6-chlorpyrid-3-yl)-isothioharnstoff werden in 50 ml Ethanol vorgelegt und mit 1,27 ml (9,1 mMol) Triethylamin und 50,0 ml Wasser versetzt. Anschließend wird der Reaktionsansatz ca 18 Stunden bei 60°C unter Schutzgas (Argon) gerührt. Nach Beendigung der Reaktion wird das Ethanol im Vakuum abdestilliert und der wässrige Rückstand dreimal gegen Chloroform geschüttelt. Die organische Phase wird nach dem Trocknen im Vakuum eingeengt und der zurückgebliebene Rückstand mittels präparativer HPLC gereinigt. Man erhält 13,5 mg (0,7 %) N-Methoxyamino-N'-(6-chlorpyrid-3-yl)-cyanoguanidin.
C₉H₁₀ClN₅OS (239,7)
LC-MS m/z (%) = 239 (M⁺)

### Ausgangsstoffe der Formel (II):

### Beispiel (II-1)

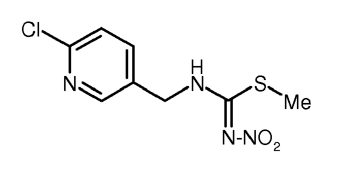

### Stufe 1

### S-Methyl-N-nitro-N'-phthaloyl-isothioharnstoff

24,7 g (0,18 mol) S-Methyl-N-nitro-isothioharnstoff (vgl. L. Fishbein, J. A. Gallaghan, J. Am. Chem. Soc. 76, 1877, 1954) werden in 345 ml Pyridin verrührt und bei 0°C bis 5°C tropfenweise mit 71,6 g (0,35 mol) Phthalsäurechlorid versetzt. Nach 30-minütigem Rühren wird wir das gesamte Reaktionsgemisch auf ein Gemisch aus Eiswasser/Salzsäure gegeben und gut verrührt. Der ausgefallene Feststoff wird abgetrennt und in heißem Ethanol verrührt.

Nach dem Abtrennen des Feststoffs erhält man 36,7 g (39 % der Theorie) S-Methyl-N-nitro-N'-phthaloyl-isothioharnstoff, der ohne weitere Reinigung für die weitere Umsetzung verwendet werden kann.

### Stufe 2

23,3 g (0.088 mol) S-Methyl-N-nitro-N'-phthaloyl-isothioharnstoff werden in 250 ml Acetonitril vorgelegt und bei 0°C bis 5°C mit einer Lösung aus 12,5 g (0,088 mol) 3-Aminomethyl-6-chlor-pyridin in 50 ml Acetonitril innerhalb von 30 Minuten versetzt. Danach rührt man die Mischung weitere 30 Minuten bei 0°C bis 5°C und ca. 18 Stunden bei Raumtemperatur (ca. 20°C). Der ausgefallenen Feststoff wird abgetrennt und in einem Gemisch aus Methanol / Essigsäureethylester (1:1) verrührt (ungelöster Rückstand: 3,9 g). Die organische Lösung wird mit Essigsäureethylester/5%iger Natronlauge extrahiert und ergibt weitere 11.3 g (49 % der Theorie) N-(6-Chlor-pyrid-3-yl-methyl)-S-methyl-N'-nitro-isothioharnstoff, der direkt für die weitere Umsetzung verwendet werden kann.

### N-Methoxy-N'-nitro-guanidin

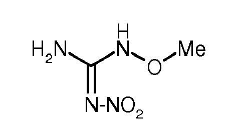

27,0 g (0,20 mol) S-Methyl-N-nitro-isothioharnstoff (vgl. L. Fishbein, J. A. Gallaghan J. Am. Chem. Soc. 76, 1877, 1954) und 16,7 g (0,20 mol) O-Methyl-hydroxylamin-hydrochlorid werden in 400 ml Wasser verrührt, mit 22,2 g (0,22 mol) Triethylamin versetzt und 16 Stunden bei 55°C gerührt. Bereits nach 2 bis 3 Stunden lässt die Mercaptanbildung nach. Danach wird der gesamte Reaktionsansatz im Vakuum bis zur Trockne eingeengt und anschließend mit einem Gemisch aus 100 ml Wasser / 200 ml Methylenchlorid verrührt.

Man erhält 7,6 g (28,4 % der Theorie) N-Methoxy-N'-nitro-guanidin als hellgelbes Pulver.

Aus der Mutterlauge können nach Zugabe von Natriumhydrogencarbonat-Lösung und Extraktion mit Methylenchlorid 3,0 g (12,5 % der Theorie) N-Methoxy-S-methyl-isothioharnstoff als Nebenprodukt isoliert werden.
C₂H₆N₄O₃ (134,0)
LC-MS m/z (%) = 135 (MH⁺, 100)
Fp.: 138-142 °C
¹H-NMR (600 MHz, DMF-d₇, δ) = 3,78 (3H, OCH₃); 8,03 (2H, br., NH₂); 11,36 (1H, NH) ppm.
¹³C mit ¹H-Entkopplung (CPD, 100 MHz, DMF-d₇, δ) = 64,5 (OCH₃); 161,4 (C=N) ppm.
¹H- /¹⁵N-Korrelation (HMBC, 600 MHz, DMF-d₇, δ) = -212,9 (NH-OCH₃) ppm.

### N-Methoxy-S-methyl-isothioharnstoff:

C₃H₈N₂OS (120,1)
LC-MS m/z (%) = 121 (MH⁺, 100)
¹H-NMR (600 MHz, DMF-d₇, δ) = 2,25 (3H, SCH₃); 3,61 (3H, OCH₃); 6,02 (2H, br., -NH₂) ppm.
¹³C mit ¹H-Entkopplung (CPD) und ¹H- mit ¹³C-korrelation (HMBC, 600 MHz, DMSO-d₆, δ) = 12,8 (SCH₃); 60,6 (OCH₃); 151,3 (C=N) ppm.
¹H- /¹⁵N-Korrelation (HMBC, 600 MHz, DMSO-d₆, δ) = -308,2 (NH₂); -86,7 (C=N) ppm.

### Beispiel (II-2)

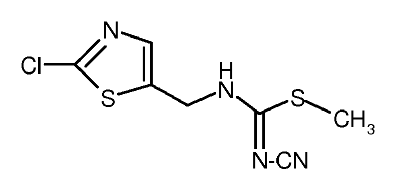

### S-Methyl-N-cyan-N'-(2-chlor-1,3-thiazol-5-yl)-isothioharnstoff

500 mg (3,36 mMol) 5-Chlormethyl-2-chlor-1,3-thiazol werden in 80 ml Ethanol verrührt und bei 0°C tropfenweise mit einer ethanolischen Lösung von 738 mg (5,0 mMol) N-Cyan-dithiocarbamidsäuredimethylester versetzt. Anschließend lässt man das Reaktionsgemisch weitere 18 Stunden bei Raumtemperatur rühren, engt den gesamten Reaktionsansatz im Vakuum ein, löst den gebildeten Rückstand in 30 ml heißem Ethanol und filtriert anschließend. Nach Abkühlung werden die Kristalle abgetrennt und auf einer Tonschale getrocknet. Man erhält 687,5 mg (75,4 %) S-Methyl-N-cyan-N'-(2-chlor-1,3-thiazol-5-yl)-isothioharnstoff.
C₇H₇ClN₄S₂ (246,7)
LC-MS m/z (%) = 247 (M⁺)
¹H-NMR (400 MHz, CD₃CN, δ) = 2,51 (3H, SCH₃); 4,63 (2H, NH-CH₂); 7,17 (2H, br., NH); 7,49 (1H, thiazol-H) ppm.

### Beispiel (II-3)

Analog erhält man S-Methyl-N-cyan-N'-(6-chlorpyrid-3-yl)-isothioharnstoff
C₉H₉ClN₄S (240,7)
LC-MS m/z (%) = 241 (M⁺)

### Beispiel A

### Myzus persicae-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Grünen Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

In diesem Test zeigen z.B. die Verbindungen der Herstellungsbeispiele I-1 und I-5 gute Wirksamkeit.

**Tabelle A**

| Pflanzenschädigende Insekten | | |
|---|---|---|
| **Myzus-Test** | | |
| Wirkstoffe | Wirkstoffkonzentration in ppm | Abtötungsgrad in % nach 6^{d} |
| 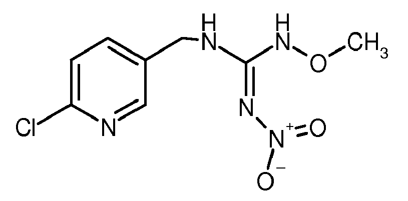 | | |
| (I-1) | 1000 | 98 |
| 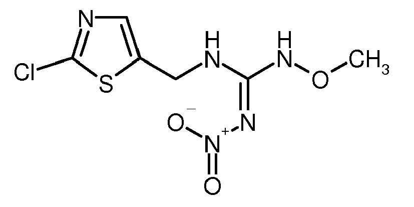 | | |
| (I-5) | 1000 | 98 |

### Beispiel B

### Myzus-Test (Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Chinakohlblattscheiben (Brassica pekinensis), die von allen Stadien der Grünen Pfirsichblattlaus (Myzus persicae) befallen sind, werden mit einer Wirkstoffzubereitung der gewünschten Konzentration gespritzt.

Nach der gewünschten Zeit wird die Wirkung in % bestimmt. Dabei bedeutet 100%, dass alle Blattläuse abgetötet wurden; 0% bedeutet, dass keine Blattläuse abgetötet wurden.

Bei diesem Test zeigt z.B. die Verbindung gemäß Herstellungsbeispiel I-2 gute Wirksamkeit.

**Tabelle B**

| Pflanzenschädigende Insekten | | |
|---|---|---|
| **Myzus-Test** | | |
| (Spritzbehandlung) | | |
| Wirkstoffe | Wirkstoffkonzentration in ppm | Abtötungsgrad in % nach 4^{d} |
| 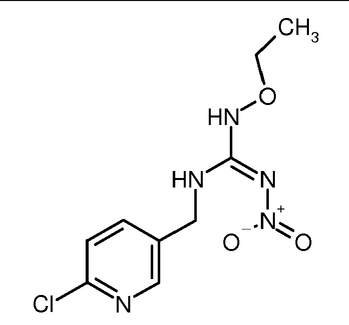 | | |
| (I-2) | 500 | 100 |

### Beispiel C

### Myzus persicae-Test (Bodenapplikation)

| | |
|---|---|
| Lösungsmittel: | 4 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Die Wirkstoffzubereitung wird mit Erde vermischt. Die angegebene Konzentration bezieht sich auf die Wirkstoffmenge pro Volumeneinheit Boden (mg/l = ppm). Man füllt den behandelten Boden in Töpfe und bepflanzt ihn mit einer Paprikapflanze (Capsicum annuum). Nach einer Woche wird mit der Grünen Pfirsichblattlaus (Myzus persicae) infiziert.

Nach der gewünschten Zeit wird die Wirkung in % bestimmt. Dabei bedeutet 100%, dass alle Blattläuse abgetötet wurden; 0% bedeutet, dass keine Blattläuse abgetötet wurden.

Bei diesem Test zeigen z.B. die Verbindungen der Herstellungsbeispiele I-1 und I-5 gute Wirksamkeit.

**Tabelle C**

| Pflanzenschädigende Insekten | | |
|---|---|---|
| **Myzus persicae-Test** | | |
| (Bodenapplikation) | | |
| Wirkstoffe | Wirkstoffkonzentration in ppm | Abtötungsgrad in % nach 7^{d} |
| 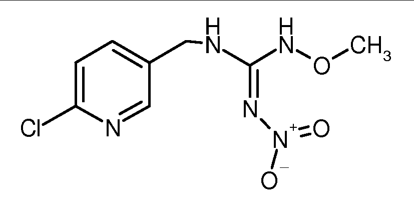 | | |
| (I-1) | 8 | 100 |
| 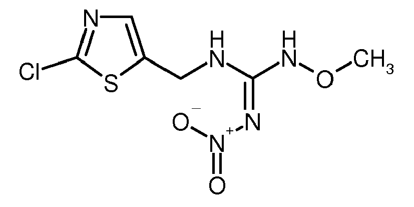 | | |
| (I-5) | 0,25 | 85 |

### Beispiel D

### Aphis gossypii-Test (Bodenapplikation)

| | |
|---|---|
| Lösungsmittel: | 4 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und der angegebenen Menge Emulgator und verdünnt das Konzentrat mit Wasser auf die gewünschte Konzentration.

Die Wirkstoffzubereitung wird mit Erde vermischt. Die angegebene Konzentration bezieht sich auf die Wirkstoffmenge pro Volumeneinheit Boden (mg/l = ppm). Man füllt den behandelten Boden in Töpfe und bepflanzt ihn mit einer Baumwollpflanze (Gossypium hirsutum). Nach einer Woche wird mit der Baumwollblattlaus (Aphis gossypii) infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z.B. die Verbindungen der Herstellungsbeispiele I-1 und I-5 gute Wirksamkeit.

**Tabelle D**

| Pflanzenschädigende Insekten | | |
|---|---|---|
| **Aphis gossypii-Test** | | |
| (Bodenapplikation) | | |
| Wirkstoffe | Wirkstoffkonzentration in ppm | Abtötungsgrad in % nach 7^{d} |
| 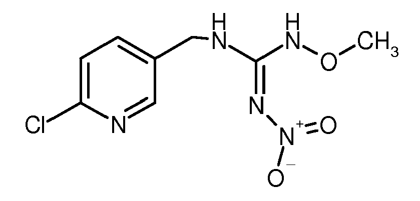 | | |
| (I-1) | 8 | 100 |
| 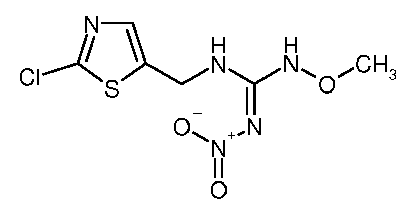 | | |
| (I-5) | 0,25 | 80 |

### Beispiel E

### Bemisia tabaci-Test (resistenter Stamm)

| | |
|---|---|
| Lösungsmittel: | 10 Gewichtsteile Aceton |
| Emulgator: | 0,2 Gewichtsteile Triton X-100 |

Zur Herstellung einer zweckmäßigen Wirkstoffzubereitung vermischt man 1 Gewichtsteil Wirkstoff mit den angegebenen Mengen Lösungsmittel und der angegebenen Menge Emulgator und verdünnt das Konzentrat mit emulgatorhaltigem Wasser auf die gewünschte Konzentration.

Blattscheiben von Baumwollpflanzen (Gossypium hirsutum) werden durch Tauchen in die Wirkstoffzubereitung der gewünschten Konzentration behandelt und nach dem Antrocknen des Belages mit Adulten der Weißen Fliege (Bemisia tabaci, resistenter Stamm) besetzt.

Nach der gewünschten Zeit wird die Abtötung in % bestimmt. Dabei bedeutet 100 %, dass alle Tiere abgetötet wurden; 0 % bedeutet, dass keine Tiere abgetötet wurden.

Bei diesem Test zeigt z.B. die Verbindung gemäß Herstellungsbeispiel I-5 gute Wirksamkeit.

**Tabelle E**

| Pflanzenschädigende Insekten | | |
|---|---|---|
| **Bemisia tabaci-Test** | | |
| (resistenter Stamm) | | |
| Wirkstoffe | Wirkstoffkonzentration in ppm | Abtötungsgrad in % nach 3^{d} |
| 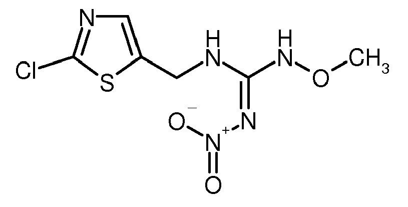 | | |
| (I-5) | 1000 | 93 |

### Beispiel F

### Myzus-Test (Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und der angegebenen Menge Emulgator und verdünnt das Konzentrat mit emulgatorhaltigem Wasser auf die gewünschte Konzentration.

Chinakohlblattscheiben (brassica pekinensis), die von allen Stadien der Grünen Pfirsichblattlaus (MYzus persicae) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

**Tabelle F**

| Pflanzenschädigende Insekten | | |
|---|---|---|
| **Myzus-Test** | | |
| (Spritzbehandlung) | | |
| Wirkstoffe | Wirkstoffkonzentration g a.i./ha | Wirkung in % nach 5^{d} |
| 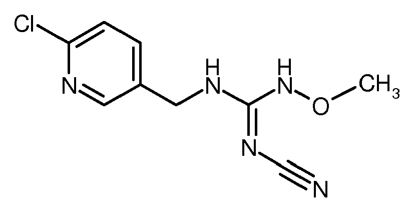 | 500 | 100 |
| (I-11) | | |
| 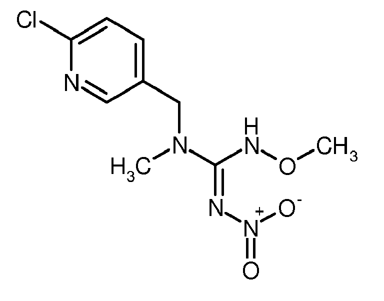 | 500 | 100 |
| (I-9) | | |

### Beispiel G

### Spodoptera frugiperda-Test

| | |
|---|---|
| Lösungsmittel: | 7 Gewichtsteile Dimethylformamid |
| Emulgator: | 2 Gewichtsteile Alkylarylpolaglykolether |

Zur Herstellung einer zweckmäßigen Wirkstoffzubereitung vermischt man 1 Gewichtsteil Wirkstoff mit den angegebenen Mengen Lösungsmittel und der angegebenen Menge Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, so lange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

**Tabelle G**

| Pflanzenschädigende Insekten | | |
|---|---|---|
| **Spodoptera frugiperda-Test** | | |
| Wirkstoffe | Wirkstoffkonzentration g a.i./ha | Abtötungsgrad in % nach 7^{d} |
| 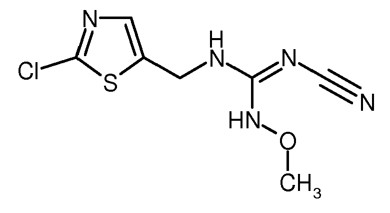 | | |
| (I-10) | 500 | 100 |

### Beispiel H

### Frankliniella occidentalis-Test

| | |
|---|---|
| Lösungsmittel: | 7 Gewichtsteile Dimethylformamid |
| Emulgator: | 10 Gewichtsteile Alkylarylpolaglykolether |

Zur Herstellung einer zweckmäßigen Wirkstoffzubereitung vermischt man 1 Gewichtsteil Wirkstoff mit den angegebenen Mengen Lösungsmittel und der angegebenen Menge Emulgator und verdünnt das Konzentrat mit emulgatorhaltigem Wasser auf die gewünschte Konzentration.

Baumwollpflanzen (Gossypium hirsutum) werden durch Spritzen mit der Wirkstoffzubereitung in der gewünschten Konzentration behandelt und mit einer gemischten Thripspopulation (Frankliniella occidentalis) infiziert.

Nach der gewünschten Zeit wird die Abtötung in % bestimmt. Dabei bedeutet 100 %, dass alle Thripse abgetötet wurden; 0 % bedeutet, dass keine Thripse abgetötet wurden.

**Tabelle H**

| Pflanzenschädigende Insekten | | |
|---|---|---|
| **Frankliniella occidentalis-Test** | | |
| Wirkstoffe | Wirkstoffkonzentration in ppm | Abtötungsgrad in % nach 14^{d} |
| 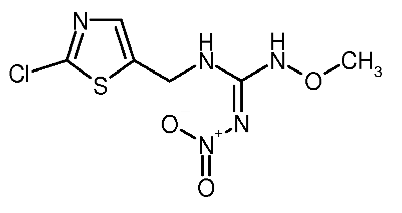 | | |
| (I-5) | 100 | 90 |

## Patentansprüche

1. Verbindungen der Formel (I) 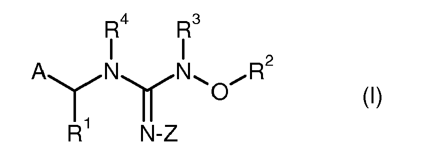 in welcher
A für gegebenenfalls substituiertes Tetrahydrofuryl steht,
R¹ für Wasserstoff oder Alkyl steht,
R² für Wasserstoff oder Alkyl steht,
R³ für Wasserstoff oder Alkyl steht,
R⁴ für Wasserstoff, Alkyl oder Cycloalkyl steht, und
Z für Cyano steht.

2. Verbindungen der Formel (I) gemäß Anspruch 1, worin
A für gegebenenfalls durch C₁-C₄-Alkyl substituiertes Tetrahydrofuryl steht,
R¹ für Wasserstoff oder Methyl, Ethyl oder n-Propyl oder iso-Propyl steht,
R² für Wasserstoff, Methyl, Ethyl, n-Propyl oder n-Butyl steht,
R³ steht bevorzugt für Wasserstoff, Methyl oder Ethyl steht,
R⁴ für Wasserstoff, Methyl, Ethyl, n- oder i-Propyl, Cyclopropyl, Cyclobutyl, Cyclopentyl oder Cyclohexyl steht und
Z für Cyano steht.

3. Verfahren zur Herstellung von Verbindungen der Formel (I) gemäß Anspruch 1, **dadurch gekennzeichnet, dass** man Isothioharnstoffe der Formel (II) 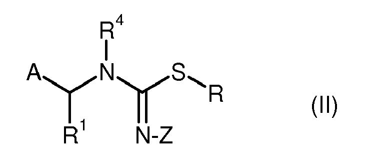 in welcher
A, R¹, R⁴ und Z die oben angegebene Bedeutung haben und
R für Alkyl steht,
mit Oxyaminen der Formel (III) 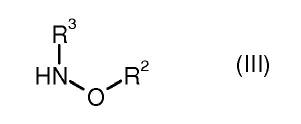 in welcher
R² und R³ die oben angebenene Bedeutung haben,
- oder mit Säureaddukten der Verbindungen der Formel (II), wie beispielsweise deren Hydrochloriden -
gegebenenfalls in Gegenwart eines basischen Reaktionshilfsmittel und gegebenenfalls in Gegenwart eines Verdünnungsmittel umsetzt.

4. Mittel, enthaltend mindestens eine Verbindung der Formel (I) gemäß Anspruch 1.

5. Verwendung von Mitteln gemäß Anspruch 4 zur Bekämpfung von tierischen Schädlingen.

6. Verfahren zur Herstellung von Mitteln gemäß Anspruch 4, **dadurch gekennzeichnet, dass** man Verbindungen der Formel (I) mit Streckmitteln und/oder oberflächenaktiven Stoffen vermischt.
